# EUROPEAN PATENT APPLICATION

(11) **EP 3 138 565 A1**
(43) Date of publication of application: **08.03.2017**
(21) Application number: 14802517.4
(22) Date of filing: 28.04.2014
(51) Int. Cl.: A61K 31/498, A61P 35/00

(54) **METHOD FOR TREATING A TUMOUR DISEASE AND METHOD FOR SELECTIVELY INHIBITING TUMOUR CELL GROWTH USING A QUINOXALINE-1,4-DIOXIDE DERIVATIVE**

(71) Applicant: OOO "A-Laboratory", Moscow 117321 (RU)
(72) Inventor: SHCHEKOTIKHIN, Andrey Egorovich, Moscow 123154 (RU); SELIVANOVA, Galina Nikolaevna, Stockholm 11360 (SE); POROIKOV, Vladimir Vasilievich, g. Zheleznodorozhny Moskovskaya obl. 143986 (RU); ZAKHAROV, Aleksey Vladimirovich, Moscow 127349 (RU); KEL, Alexander Eduardovich, g. Berdsk Novosibirskaya obl. 633001 (RU); KUTCHEROV, Vladimir Georgievich, Moscow 117321 (RU)
(74) Representative: Hartig, Michael
(86) International application number: PCT/RU2014/000310
(87) International publication number: WO 2015/167350

(57) **Abstract**

The present invention relates to the fields of pharmacology and medicine, in particular, to the medical application of 6,7-difluoro-3-phenyl-2-cyanoquinoxaline 1,4-dioxide of formula **I:** 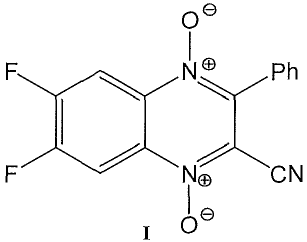 for inhibition of tumor cell growth or treatment of tumor diseases.

## Description

### Field of the invention

The invention relates to the fields of pharmacology and oncology; particularly, it relates to a new medical application of a derivative of quinoxaline 1,4-dioxide for treatment of malignant tumors.

### Background of the invention

Derivatives of quinoxaline 1,4-dioxide are known to possess a wide spectrum of biological activity. It is well known that the compounds of the series are used in the development of chemotherapy agents. Derivatives of quinoxaline 1,4-diozide have been used in medicine and veterinary as antibacterial agents (the dioxidin, quidoxin, and carbadox drugs) to treat severe forms of infectious diseases. In recent decades, candidate compounds for treatment of fungal infections [Carta A, et al. Eur J. Med Chem., 2002, 37, 355], tuberculosis [Vicente E. et al. Bioorg. Med. Chem., 2009, 17, 385], malaria [Zarranz B. et al. Arzneim Forsch/Drug Res., 2005, 55, 754; Vicente E. et al. Eur. J. Med. Chem., 2008, 43, 1903; Barea C. et al. Bioorg. Med. Chem. Lett., 2011, 21, 4498], and a number of tropical parasitic diseases [Vicente E. et al., Bioorg. Med. Chem. Lett., 2010, 20, 4831; Gerpe A. et al., Eur. J. Med. Chem., 2010, 45, 2154; Barea C. et al, Molecules 2012, 17, 9451; Benitez D. et al., Med Chem Res., 2012, 21, 1439; Barea C. et al, Molecules 2013, 18, 4718] have been found among these substances.

Besides, it is known that quinoxaline 1,4-dioxides are able to induce death of tumor cells. Works on the search for cytotoxic compounds were the most active among the 3-amino-2-cyanoquinoxaline 1,4-dioxides, which are analogues of an antitumor drug tirapamazin. Among the series, compounds (for example, a derivative of formula **1)** that act more selectively upon tumor cells under hypoxia than tirapamazin were discovered and thus some of them were patented as antitumor agents and radiosensibilizators [GB 22970889], and cytotoxic properties of the compounds of this type have been described in a number of publications [Mongeetal A. et al. J. Med. Chem., 1995, 38, 1786; Mongeetal A. et al. J. Med. Chem., 1995, 38, 4488; Crespo F.J.M. et al. J. Heterocyclic. Chem., 1996, 33, 1671; Ortega M.Á. et al. Eur. J. Med. Chem., 2000, 35, 21; Ismail M.M.F. et al. Eur. J. Med. Chem., 2010, 45, 2733].

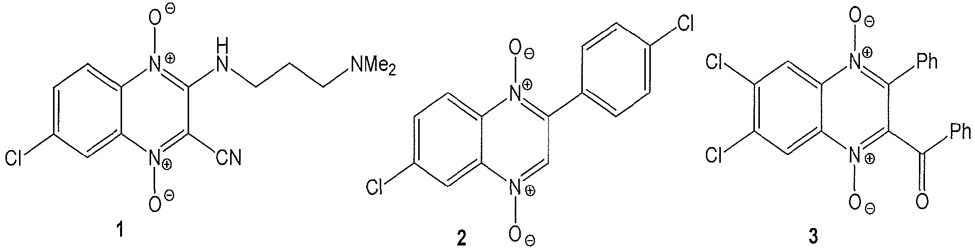

Some 2-arylquinoxaline 1,4-dioxides (for example, the compound of formula **2** [Amin K.M. et al. Bioorg. Med. Chem., 2006, 14, 6917]) and 2-acylquinoxaline 1,4-dioxides (for example, the compound of formula **3** [Gali-Muhtasib H.U. et al. Oncol. Rep., 2001, 8, 679]) are also able to cause death of tumor cells under conditions of hypoxia while producing practically no effect on cells under conditions of normoxia. Besides, it is known that some 2-acyl- and 2-alkylsulfonyl derivatives of quinoxaline 1,4-dioxide are characterized by antitumor activity in vivo [Solano B. et al. Journal of Medicinal Chemistry, 2007, 50, 548; Weng Q. et al. Invest. New Drugs, 2011, 29, 1177].

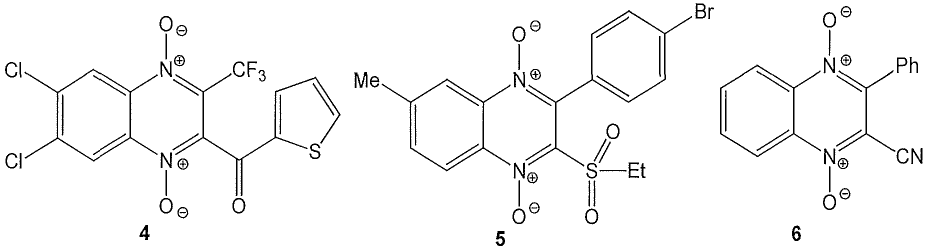

Relatively recently the ability to inhibit hypoxic tumor cells has been revealed for 3-aryl-2-cyanoquinoxaline 1,4-dioxides (for example, the compound of formula **6** [Hu Y. et al. Molecules 2012, 17, 9683]). According to the authors of the present invention, this publication may be viewed as the closest analogue of the invention claimed, however the derivatives of quinoxaline 1,4-dioxide described in the publication possess low activity with respect to tumor cells at normoxia state.

It is well known that the efficiency of antitumor agents is often limited by the multiple drug resistance (MDR) of the tumor cells developed in response to chemotherapy or acquired in the course of disease progression [Shtil A.A.J. Hematother. Stem Cell Res., 2002, 11, 437]. Expression of ATP-binding cassette transporters, for example p-glycoprotein (p-gp), on surface of tumor cells [Ambudkar S.V., et al. Oncogene 2003, 22, 7468] is one of the most common mechanisms of resistance developing in tumor cells in response to chemotherapy [Knez L., et al. Lung Cancer. 2011, 72, 271]. Therefore, one of the ways to increase the efficiency of chemotherapy is the search for new drugs able to affect tumor cells with MDR induced by chemotherapy agents.

The aim of the invention was to identify a selective cytotoxic compound relating to the derivatives of 3-aryl-2-cyanoquinoxaline 1,4-dioxides applicable in therapy of tumor diseases, including tumors resistant to other drugs.

### Disclosure of the invention

The authors of the invention have conducted intense studies including the virtual screening, synthesis of a series of polysubstituted 2-cyanoquinoxaline 1,4-dioxides, and investigation of their biological properties. As a result, an unexpected property of a previously described 6,7-difluoro-3-phenyl-2-cyanoquinoxaline 1,4-dioxide **(7),** that is, the fact that the compound of the formula **7** surpasses considerably all the cytotoxic 3-aryl-2-cyanoquinoxaline 1,4-dioxides described in the literature thus far by the activity against tumor cells, has been revealed. For example, the activity of the compound of formula **7** surpasses that of the compound of formula **6** [Hu Y. et al. Molecules, 2012, 17, 9683] with respect to tumor cells by two orders of magnitude.

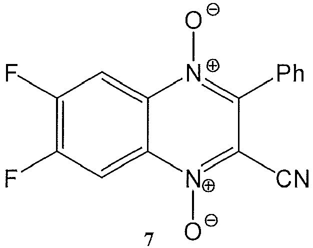

Authors of the present invention also demonstrated that the compound of formula **7** at submicromolar concentrations induced apoptosis in all studied types of human tumor cells of various genesis under conditions of normoxia, and also induced death of a number of sublines with multiple drug resistance caused by the expression of ATP-binding cassette transporters, for example, p-glycoprotein (p-gp) on surface of tumor cells.

Besides, the derivative of the formula **7** possesses high selectivity of the cytotoxic effect with respect to tumor cells, since it causes practically no death of normal cells, for example, fibroblasts, at concentrations of up to 6 µM. Moreover, 6,7-difluoro-3-phenyl-2-cyanoquinoxaline 1,4-dioxide possesses a reliable antitumor effect on animal tumor models.

Therefore, the invention relates to the method of selective inhibition of tumor cells that comprises contacting of said tumor cells and the efficient quantity of 6,7-difluoro-3-phenyl-2-cyanoquinoxaline 1,4-dioxide of formula **7:**

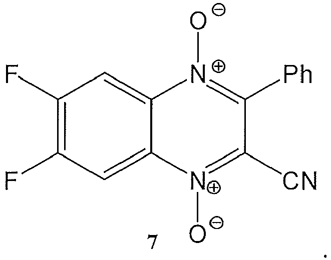

According to one of the preferred embodiments, said tumor cells are characterized by drug resistance to other antitumor compounds.

Besides, the invention relates to the method of treatment of tumor disease of a patient in need thereof that comprises administering to said patient a therapeutically efficient quantity of 6,7-difluoro-3-phenyl-2-cyanoquinoxaline 1,4-dioxide of formula 7 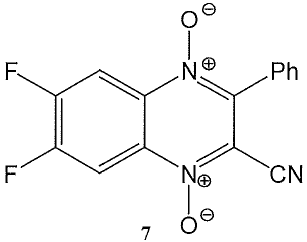 to said patient.

According to one of the preferred embodiments, said tumor disease is characterized by drug resistance to other antitumor compounds.

The diseases that are subject of treatment according to the present invention are characterized by increased cell growth rate (that is, hyperproliferation) and are, above all, tumor diseases of various geneses, including but not limited to carcinomas, leukemias, lymphomas, melanomas, sarcomas, glyomas, etc. In particular, in a preferred embodiment, tumor disease to be treated by the method according to the invention can be selected from a group including malignant neoplasms of breast, uterus, intestines, lung, bladder, testicles, prostate, ovary, thyroid gland, or bone, soft tissue sarcoma, melanoma, or leukemias. In an especially preferred embodiment, quinoxaline 1,4-dioxide can be used for treatment of tumor diseases resistant to doxorubicin, taxol, vinca alkaloids, or other chemotherapeutics.

The term "patient" as used herein refers to a man or animal in need of treatment without specification of age or gender.

The term "efficient quantity" of "therapeutically efficient quantity" of a compound as used herein refers to the amount of 6,7-difluoro-3-phenyl-2-cyanoquinoxaline 1,4-dioxide (compound of formula **7)** that suppresses considerably proliferation of tumor cells and is efficient in preventing, relieving, or alleviating the disease symptoms or increasing of survival of the subject treated. Determination of the therapeutically efficient quantity is referred to the competence of the specialist in the field. Therapeutically efficient quantity or dosage of the compound proposed in the invention can change within wide ranges and can be determined by a method known in the field.

For a certain recipient, the relevant therapy line is selected taking into account its individual need and the opinion of a physician who administers the pharmaceutical compositions to the patient or prescribes administration of pharmaceutical compositions. Daily dose of a therapeutic agent may be administered once in a single dose or multiple times in divided doses administered at appropriate intervals of time or, in the case of parenteral administration, it may be administered by means of continuous infusion. Therefore, the required quantity, for example, the required therapeutically efficient quantity, is determined by a specialist in the given field of medicine. For example, the dose of the therapeutic agent can vary in function of age, body weight, or conditions in the range from approximately 1 mg to approximately 100 mg 6,7-difluoro-3-phenyl-2-cyanoquinoxaline 1,4-dioxide (7) per kilogram body weight of the recipient per day.

The compound of formula **7** used according to the present invention can be obtained by various methods, for example, through Beirut reaction described in the literature [Kotovskaya S.K. et al. Russ. J. Org. Chem., 2002, 38, 1046], or by a method described in the example below. The following examples demonstrate, but in no way limit, the efficiency of the present invention.

### Example 1

### Synthesis of 6,7-difluoro-3-phenyl-2-cyanoquinoxaline 1,4-dioxide

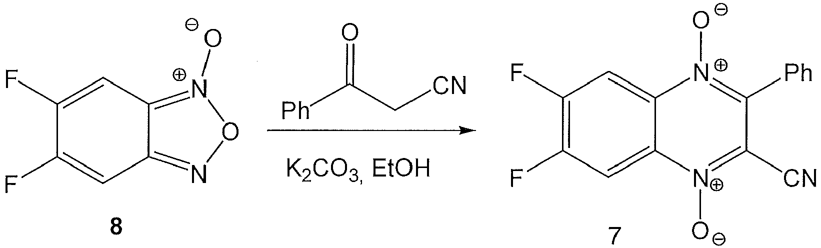

Mixture of 600 mg (0.34 mmol) 5,6-difluorobenzofuroxane **8,** 730 mg (0.45 mmol) benzoylacetonitrile, and 60 mg (0.05 mmol) anhydrous potassium carbonate in 200 mL ethanol was stirred for 12 h. The precipitate formed was filtered off, washed with ethanol, and recrystalized from dioxane. Yield of quinoxaline 1,4-dioxide of formula **7** was 453 mg (43%). mp 220-222°C. HPLC (Kromasil 100-5 C18 column, 4.6 × 250 mm (Akzo Nobel); eluents: A, H₃PO₄ (0.01 M); B, MeCN; gradient of B 40 → 65% (20 min); LW = 273 nm): *t_{R} =* 15.2 min, 99.2% purity. UV spectrum, nm (EtOH): 237, 273, 296, 379, 423. ¹H NMR spectrum (400 MHz, DMSO-d₆), δ, ppm: 7.70 (2H, m, H-5, H-8); 6.79 (2H, m, C₆H₅); 6.70 (3H, m, C₆H₅). ¹³C NMR spectrum, δ, ppm (J, Hz): 154.0 (dd, *J¹* = 259.7, *J²* = 15.5, 6-CF); 152.9 (dd, *J¹* = 258.5, *J²* = 15.7, 7-CF); 143.45 (3-C); 136.82 (d, *J* = 9.3, 10-C); 134.53 (d, *J =* 9.2, 9-C); 131.34 (2 x 3'-C); 130.13 (2 x 3'-C); 128.69 (1'-C); 127.37 (4'-C); 120.96 (CN); 110.89 (2-C); 109.90 (d, *J =* 28.3, 8-CH); 109.07 (d, *J =* 28.8, 5-CH). IR spectrum, cm⁻¹: 2924, 1614, 1515, 1466, 1358, 1337, 1315, 1278, 1207, 1173, 1128, 979, 925, 775, 751, 696. Found: *m*/*z* (ESI), 300.0567. [M+H]⁺. C₁₅H₈F₂N₃O₂⁺. Calcd: 300.0579.

### Example 2

### The effect of 6,7-difluoro-3-phenyl-2-cyanoquinoxaline 1,4-dioxide on tumor growth

Comparative study of cytotoxicity of 6,7-difluoro-3-phenyl-2-cyanoquinoxaline 1,4-dioxide (compound **7** obtained according to a method described in the Example 1) and the previously known 6,7-nonsubstituted analogue **6** (obtained earlier by a method described by [Hu Y. etal. Molecules 2012, 17, 9683]) was performed in in vitro tests on cells of human solid tumors, as well as normal human embryo lung fibroblasts (HELF) to control selectivity. Data on the antiproliferative activity (IC₅₀, concentration of 50% growth inhibition) are presented in Table 1. IC₅₀ values were determined by the MTT test according to a protocol described in the literature [Mossman T.J. Immunol. Methods, 1983, 65, 55].

**Table 1. Antiproliferative activity (IC₅₀, µM) of quinoxaline 1,4-dioxides 6 and 7 with respect to human solid tumor cells and non-tumor human embryo lung fibroblasts (HELF)**

| **Compound ID** | **Structure** | **IC₅₀, µM** | | |
|---|---|---|---|---|
| | | **HCT116** | **MSF7** | **HEFL** |
| **7** | 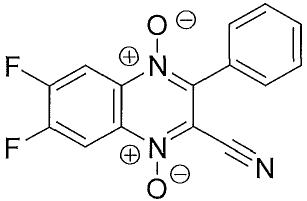 | 0.60 ± 0.03 | 0.1 ± 0.02 | 44 ± 1.9 |
| **6** | 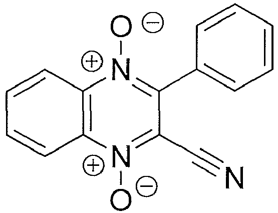 | 8.4 ± 0.8 | 15.5 ± 0.98 | 25.2 ± 5.1 |

As follows from the data, 6,7-difluoro-3-phenyl-2-cyanoquinoxaline 1,4-dioxide of formula **7** inhibits growth of all tested tumor cell lines at concentrations that are two orders of magnitude lower than those of the closest analogue of the formula **6;** cytotoxic properties of the latter one have been previously described in the literature [Hu Y. etal. Molecules 2012, 17, 9683]. Besides, cytotoxicity of the cyanoquinoxaline 1,4-dioxide 7 with respect to tumor cells of different genesis was 200-300 higher than against the non-tumor human fibroblast cells, which evidences its high selectivity.

### Example 3

### The effect of 6,7-difluoro-3-phenyl-2-cyanoquinoxaline 1,4-dioxide on growth of tumor cells with multiple drug resistance

Comparative study of cytotoxicity of 6,7-difluoro-3-phenyl-2-cyanoquinoxaline 1,4-dioxide (compound of formula **7),** which is the subject if the present invention, was performed also on a culture of tumor lymphocytic leukemia K562 cells and its resistant subline K562/4 expressing p-glycoprotein. Expression of such ATP-dependent transporters on cell surface is one of the most common mechanisms of the multidrug resistance development in tumors [Shtil A.A., Grinchuk T.M., et. al. Int. J. Oncol., 2000, 17, 387]. Data of these studies, including the IC₅₀ values of reference compounds, the previously described quinoxaline 1,4-dioxide of formula **6,** and doxorubicin, as well as the resistance index value (RI is the ratio between the IC₅₀ values of the K562/4 subline and IC₅₀ of the K562 line) are presented in Table 2.

As follows from the data, 6,7-difluoro-3-phenyl-2-cyanoquinoxaline 1,4-dioxide (compound of formula **7)** described in the Example 1, surpasses the reference preparation, doxorubicin, almost 50-fold and the closest analogue of formula **6,** 100-fold, by the activity toward K562/4 lymphocytic leukemia cells with multiple drug resistance caused by p-gp transporter expression. Besides, 6,7-difluoro-3-phenyl-2-cyanoquinoxaline 1,4-dioxide (compound of formula **7)** exhibits similar activity toward cells of both the K562 line and its resistant subline, K562/4. Therefore, 6,7-difluoro-3-phenyl-2-cyanoquinoxaline 1,4-dioxide is characterized by almost 50-fold lower resistance index (RI) than the antitumor antibiotics doxorubicin used in clinics.

**Table 2. Antiproliferative activity (IC₅₀, µM) of quinoxaline 1,4-dioxides 6 and 7 and of the reference compound doxorubicin toward K562 leukemic cells and the K562i/4 subline with multidrug resistance caused by the expression of p-gp transporter**

| **Compound ID** | **Structure** | **IC₅₀, µM** | | **RI*** |
|---|---|---|---|---|
| | | **K562** | **K562/4** | |
| **7** | 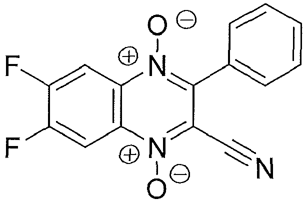 | 0.15 ± 0.02 | 0.30 ± 0.05 | 2.0 |
| **6** | 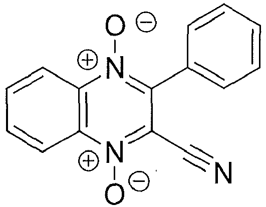 | 5.20 ± 0.78 | 33.0 ± 4.0 | 6.3 |
| Doxorubicin | 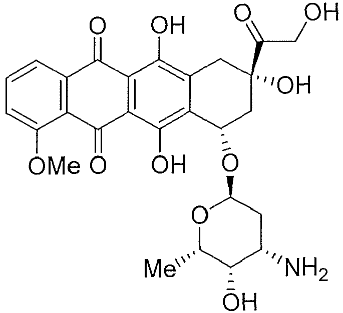 | 0.18 ± 0.03 | 18.4 ± 2.2 | 102.2 |

| | | | | |
|---|---|---|---|---|
| *RI, resistance index; RI = IC₅₀(K562/4)/IC_{50(K562)}· | | | | |

## Claims

1. The method of selective inhibition of tumor cell growth that comprises contacting of said tumor cells with the efficient quantity of 6,7-difluoro-3-phenyl-2-cyanoquinoxaline 1,4-dioxide of formula **I:**

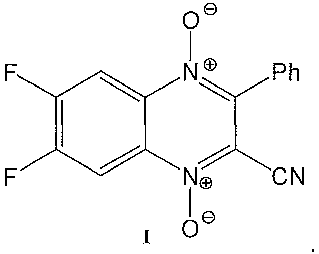

2. The method of claim 1, wherein said cells are **characterized by** drug resistance to other antitumor compounds.

3. The method of treatment of a tumor disease in a patient in need thereof that comprises administering a therapeutically efficient quantity of 6,7-difluoro-3-phenyl-2-cyanoquinoxaline 1,4-dioxide of formula **I** to said patient.

4. The method of claim 3, wherein said tumor disease is **characterized by** drug resistance to other antitumor compound.
